# FASCICULE DE BREVET EUROPEEN

(11) **EP 2 083 895 B1**
(45) Date de publication et mention de la délivrance du brevet: **04.01.2017**
(21) Numéro de dépôt: 07858665.8
(22) Date de dépôt: 06.11.2007
(51) Int. Cl.: A61M 13/00

(54) **DISPOSITIF D'INSUFFLATION DE GAZ PERMETTANT UNE UTILISATION OPTIMALE ET SECURITAIRE DES RECIPIENTS DE GAZ SOUS PRESSION**
GASBLASEVORRICHTUNG FÜR OPTIMALE UND SICHERE VERWENDUNG VON UNTER DRUCK STEHENDEN GASGEFÄSSEN
GAS BLOWING DEVICE FOR AN OPTIMAL AND SAFE USE OF PRESSURISED GAS VESSELS

(30) Priorité: 08.11.2006 FR 0654787
(43) Date de publication de la demande: 05.08.2009
(73) Titulaire: Sopro, 13705 La Ciotat Cedex (FR); Université Pierre et Marie Curie (Paris 6), 75252 Paris Cedex 05 (FR)
(72) Inventeur: SEZEUR, Alain, 94230 Cachan (FR); DROMIGNY, Fabien, 34120 Nezignan L'eveque (FR); SANDRAZ, Jean-Rémi, 13600 La Ciotat (FR); AOUSSAT, Améziane, 92120 Montrouge (FR)
(74) Mandataire: Domange, Maxime
(86) Numéro de dépôt international: PCT/FR2007/052303
(87) Numéro de publication internationale: WO 2008/056082

(56) Documents cités:
- WO-A-94/01154
- DE-A1- 2 611 698
- US-A- 5 423 741
- US-A1- 2005 222 491
- US-A1- 2005 222 534
- US-B1- 6 299 592

## Description

### Arrière-plan de l'invention

La présente invention se rapporte au domaine général des insufflateurs de gaz médicaux, notamment ceux utilisés en chirurgie. La présente invention concerne plus précisément le contrôle de la quantité de gaz restant dans un récipient sous pression connecté à un dispositif d'insufflation passif ou actif, classiquement une bouteille de gaz, liquéfié ou non.

Le but d'un tel contrôle est que le changement de bouteille soit effectué de manière suffisamment anticipée pour que l'utilisation de l'insufflation ne doive pas être interrompue brutalement et inopportunément.

Un tel contrôle est donc indispensable pour éviter l'arrêt inopportun et tout à fait préjudiciable de l'insufflation au milieu d'une intervention ou d'une session de thérapie. Actuellement, la quantité de gaz restante est classiquement contrôlée par un contrôle de la pression au sein de la bouteille. Il existe ainsi des alertes pour signaler une pression inférieure à un seuil d'alerte, par exemple 10 bars.

Cependant des bouteilles de formats divers et contenant donc des quantités et des natures de gaz différentes pour une même pression interne peuvent être connectées au dispositif d'insufflation.

Dans le cas de l'utilisation de bouteilles de faible contenance, l'alerte correspondra approximativement à un volume restant trop faible pour réaliser ou terminer une intervention ou une session de thérapie.

Mais, pour les bouteilles de grande contenance, les alertes de fin de bouteilles sont déclenchées par un seuil de pression alors qu'il existe encore suffisamment de gaz pour continuer une voire plusieurs interventions ou session(s) thérapeutique(s) sans risque bien que la pression d'alerte, généralement faible, soit atteinte. Bien souvent, la quantité de gaz non utilisée aurait permis de réaliser une voire plusieurs autres interventions ou sessions thérapeutiques. A contrario, pour une petite bouteille, l'alerte est donnée tardivement ne laissant que peu de temps pour changer la bouteille avant l'arrêt de l'insufflation.

Des dispositifs médicaux d'insufflation de gaz selon l'état de la technique sont connus des documents US 2005/222491 A1, US 2005/222534 A1, US-B1-6 299 592, WO 94/01154 A, US-A-5 423 741 et DE 26 11 698 A1.

### Objet et résumé de l'invention

La présente invention a donc pour but principal de pallier cet inconvénient. L'invention concerne en particulier le contrôle du contenu de bouteilles de gaz sous pression, voire liquéfié, en fin d'utilisation, à savoir, en particulier, quand le gaz est totalement en phase gazeuse dans la bouteille.

L'invention propose ainsi un dispositif médical d'insufflation de gaz comprenant, au moins, un circuit de sortie du gaz, un connecteur pour connecter un récipient sous pression dans lequel le gaz est conditionné, un détendeur placé entre le connecteur et le circuit de sortie du gaz, un capteur haute pression placé entre le connecteur et le détendeur, un débitmètre placé en sortie du détendeur, et un microprocesseur relié au moins au capteur haute pression pour recevoir des signaux représentatifs de la pression observée au niveau du connecteur et au débitmètre pour recevoir des signaux représentatifs du débit en sortie du détendeur, caractérisé en ce que le microprocesseur comprend des moyens pour évaluer un volume de gaz après détente restant en fonction des signaux reçus en provenance du capteur haute pression et du débitmètre lors d'une détente du gaz, ces moyens d'évaluation comportant des moyens pour évaluer le volume de gaz après détente restant comme étant le rapport du volume détendu obtenu à partir du débit mesuré par le débitmètre pendant la détente et de la durée de celle-ci sur la chute de pression engendrée par cette détente et mesurée au niveau du capteur haute pression multiplié par la pression restante mesurée par le capteur haute pression au terme de la détente.

Avec un tel dispositif, l'évaluation du volume de gaz après détente restant permet de prendre la décision de changer la bouteille quand il ne reste plus un volume suffisant de gaz pour réaliser une intervention ou une session de thérapie. Le changement de bouteille n'est alors pas décidé par rapport à un seuil de pression comme cela est le cas jusqu'à maintenant.

En particulier, l'invention permet d'utiliser le gaz restant dans des bouteilles de grande contenance pour lesquelles la pression observée quand il ne restera réellement plus assez de gaz pour réaliser une intervention ou une session de thérapie sera bien inférieure à la pression seuil.

Ainsi, l'évaluation du volume après détente restant est au minimum une sécurité complémentaire pour les petites bouteilles et au mieux une source d'économie.

Selon une caractéristique de l'invention, les moyens d'évaluation évaluent le volume de gaz après détente restant comme étant le rapport du volume détendu obtenu à partir du débit et de la durée de celle-ci sur la chute de pression engendrée par la détente et mesurée au niveau du capteur multiplié par la pression restante mesurée par le capteur au terme de la détente.

Un tel calcul présente l'avantage d'être simple à mettre en oeuvre même s'il comporte des approximations, notamment au niveau de l'influence de la température. Cette caractéristique suppose cependant que le débitmètre, dans lequel le gaz est encore à une pression intermédiaire entre la pression dans la bouteille et la pression atmosphérique, réalise une mesure du volume détendu à la pression atmosphérique. C'est généralement le cas pour tous les débitmètres utilisés dans ce domaine. Dans le cas contraire, cela introduit un simple facteur dans le calcul.

Selon un mode de réalisation particulier de l'invention, les moyens d'évaluation réalisent un calcul du volume détendu à partir du débit observé et de la durée de la détente au terme de la chute d'un intervalle de pression choisi préalablement.

Avec une telle caractéristique, une chute de pression choisie préalablement est un intervalle constant pour lequel est calculé le volume détendu. En fonction de ce volume détendu mesuré lors de la chute de pression préalablement choisie est alors évalué le volume après détente restant.

Selon un autre mode de réalisation particulier de l'invention, les moyens d'évaluation réalisent la mesure d'une chute de pression au terme de la détente d'un volume détendu choisi préalablement.

Avec une telle caractéristique, le volume détendu est choisi préalablement et c'est la chute de pression engendrée par la sortie de ce volume détendu de gaz qui permet l'évaluation du volume après détente restant.

Ainsi, lors de l'utilisation du dispositif, le calcul du volume détendu ou la mesure de la chute de pression peuvent être effectués respectivement à chaque chute d'un intervalle de pression choisi ou au terme de chaque détente successive d'un volume détendu choisi.

Le contrôle réalisé selon l'invention est particulièrement fiable dans les cas où le gaz est entièrement en phase gazeuse, c'est-à-dire généralement en fin d'utilisation d'une bouteille. Cependant, lorsque le gaz est encore au moins partiellement en phase liquide, la quantité de gaz restant en terme de volume n'est pas aisément mesurable. En effet, la pression au sein du récipient est alors très variable en fonction de la température ambiante. La pression peut ainsi varier entre 50 et 65 bars, aux températures ambiantes habituellement observées dans les blocs chirurgicaux en zone tempérée, c'est-à-dire entre 16 et 20°C. Bien souvent la quantité de gaz dans la bouteille est alors suffisante mais ce n'est pas toujours le cas pour de petites bouteilles.

En outre, il faut ajouter le fait que la détente du gaz provoque un refroidissement de celui-ci. Cela a pour conséquence directe de faire également chuter la pression. L'utilisation de la pression alors observée à la sortie de la bouteille introduit donc une erreur dans le calcul du volume restant.

Un mode de réalisation particulier de l'invention résout ce problème en incluant une sonde de température apte à mesurer la température du gaz sortant de la bouteille et relié au microprocesseur, le microprocesseur étant apte à utiliser cette mesure de température pour assurer la fiabilité du volume de gaz après détente restant évalué.

Avec une telle caractéristique, le contrôle du volume restant après détente reste possible même pour de petites bouteilles ou pour des gaz présentant des phases liquides pour des pressions relativement faibles.

Avantageusement, le dispositif est alors tel que, lorsque qu'une modification de la température supérieure à une limite donnée est observée lors d'une détente servant à l'évaluation du volume après détente restant, le microprocesseur déclenche une action choisi parmi les suivantes : alerte, non affichage du volume après détente restant, détermination d'un facteur correctif en fonction de la modification de température et utilisation de ce facteur correctif dans l'évaluation du volume après détente restant.

Selon une caractéristique avantageuse, les moyens d'évaluation sont activés lorsque le capteur mesure une pression inférieure à un seuil donné.

Au vu de ce qui précède et en l'absence d'utilisation d'une sonde de température, un tel seuil correspond avantageusement à la pression pour laquelle on sait, qu'aux températures habituelles, le gaz contenu dans le récipient est entièrement en phase gazeuse. Par exemple dans le cas du dioxyde de carbone, une telle pression est de 49 bars.

Cela évite que les moyens d'évaluation fonctionnent lorsque le gaz est encore au moins partiellement liquéfié dans la bouteille et qu'il n'existe pas de moyens correctifs ou signalétiques des variations de température.

Selon une caractéristique additionnelle du dispositif de l'invention, en dehors de l'utilisation du dispositif pour l'insufflation, les moyens d'évaluation sont avantageusement activables ponctuellement par déclenchement d'une détente prédéfinie par une chute de pression donnée ou par un volume de gaz détendu donné.

Selon une caractéristique avantageuse, le dispositif comprend des moyens d'activation ponctuelle des moyens d'évaluation manuels ou fonctionnant par réception d'un signal. Les moyens d'activation manuelle sont avantageusement un bouton placé sur le dispositif et destiné à être pressé par un opérateur ou utilisateur. Le signal reçu par les moyens de réception de signal peut être un signal provenant d'une télécommande ou un signal vocal.

Avec de telles caractéristiques, il est possible de déclencher volontairement une évaluation du volume restant en dehors de toute utilisation du dispositif. Cela est particulièrement intéressant à la mise sous tension du dispositif, par exemple par une infirmière d'un bloc opératoire qui peut alors décider de changer ou non directement la bouteille en fonction du nombre d'interventions à réaliser et du volume après détente restant évalué. Ce mode de réalisation particulier de l'invention permet donc de proposer un test de volume après détente restant avant l'utilisation du dispositif pour une ou plusieurs interventions. Cela est très utile en milieu chirurgical ou dans les services de soins ou à domicile où les décisions sont généralement à prendre rapidement et de manière sécurisée.

L'utilisation d'une commande vocale est notamment particulièrement avantageuse dans le cadre où le dispositif est utilisé en thérapie chez un malade invalide et inapte à appuyer sur un bouton.

Avantageusement, le volume de gaz après détente restant est affiché sur des moyens d'affichage reliés au microprocesseur.

Un tel affichage est utile pour informer l'opérateur du dispositif d'insufflation pour qu'il connaisse, instantanément et antérieurement à la mise en route de l'utilisation, la quantité de gaz dont il dispose pour une utilisation du dispositif en toute sécurité.

Selon une caractéristique additionnelle du dispositif de l'invention, les moyens d'évaluation permettent la détection du type de récipient connecté en fonction du calcul du rapport du volume détendu obtenu à partir du débit et de la durée de celle-ci sur la chute de pression engendrée par la détente et mesurée au niveau du capteur.

Une telle caractéristique additionnelle est utile pour l'information de l'opérateur qui connaît alors, de manière assurée, la capacité de la bouteille dont il peut encore disposer dès que le gaz qui y est contenu est en phase gazeuse.

Une telle détection du type de bouteille connectée remplace toute sélection manuelle et, par conséquent, potentiellement source d'erreur prévue dans l'art antérieur pour signaler le type de bouteille connectée.

Avantageusement, le type de récipient connecté détecté est affiché sur les moyens d'affichage.

L'invention concerne aussi un procédé apte à être implémenté dans un microprocesseur d'un dispositif d'insufflation selon l'invention, pour évaluer un volume de gaz après détente restant lors d'une détente du gaz contenu dans un récipient sous pression connecté au dispositif d'insufflation, comprenant les étapes de déclenchement d'une détente, réception de signaux représentatifs de la pression au sein du récipient en provenance du capteur haute pression, réception de signaux représentatifs du débit en sortie du détendeur en provenance du débitmètre, et évaluation du volume de gaz après détente restant comme étant le rapport du volume détendu obtenu à partir du débit mesuré par le débitmètre pendant la détente et de la durée de celle-ci sur la chute de pression engendrée par cette détente et mesurée au niveau du capteur haute pression multiplié par la pression restante mesurée par le capteur haute pression au terme de la détente.

Selon une implémentation préférée, les différentes étapes du procédé sont déterminées par des instructions de programmes d'ordinateurs.

En conséquence, l'invention vise aussi un programme d'ordinateur sur un support d'informations, ce programme étant susceptible d'être mis en oeuvre dans un microprocesseur, ce programme comportant des instructions adaptées à la mise en oeuvre des étapes du procédé selon l'invention.

Ce programme peut utiliser n'importe quel langage de programmation, et être sous la forme de code source, code objet, ou de code intermédiaire entre code source et code objet, tel que dans une forme partiellement compilée, ou dans n'importe quelle autre forme souhaitable.

L'invention vise aussi un support d'informations lisible par un microprocesseur, et comportant des instructions d'un programme d'ordinateur tel que mentionné ci-dessus.

Le support d'informations peut être n'importe quelle entité ou dispositif capable de stocker le programme.

Alternativement, le support d'informations peut être un circuit intégré dans lequel le programme est incorporé, le circuit étant également adapté pour exécuter ou pour être utilisé dans l'exécution du procédé en question.

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif.

Sur les figures :
- la figure 1 représente schématiquement un dispositif médical d'insufflation de gaz selon l'invention,
- la figure 2 est un organigramme illustrant un procédé selon l'invention,
- la figure 3 est une illustration des moyens d'affichage dans un mode de réalisation préféré de l'invention,
- la figure 4 est un diagramme pression-température correspondant au dioxyde de carbone.

### Description détaillée d'un mode de réalisation

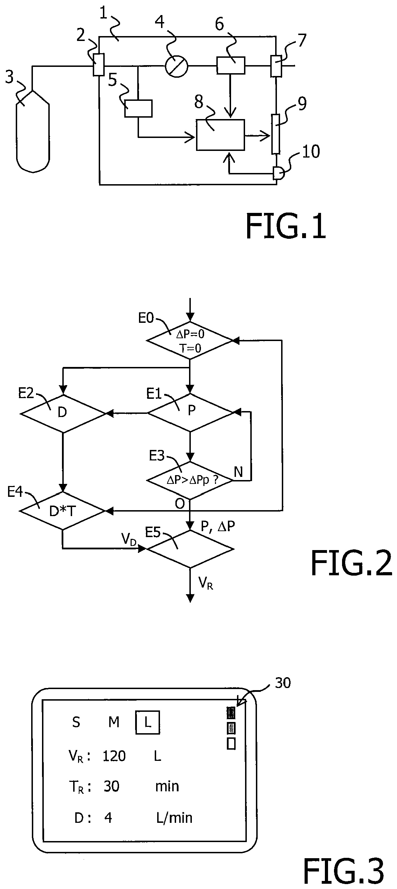

La figure 1 représente schématiquement un dispositif d'insufflation 1 selon l'invention. Un tel dispositif 1 comprend un connecteur 2 afin d'être connecté à un récipient sous pression 3, généralement une bouteille de gaz liquéfié. Comme on a vu précédemment, de telles bouteilles de gaz liquéfié existent en plusieurs formats, l'invention permettant d'optimiser l'utilisation de telles bouteilles de formats différents.

Le dispositif d'insufflation 1 comprend en outre un détendeur 4, à la sortie duquel le gaz est à une basse pression fixe et constante, par exemple 3 ou 3,5 bars. Il comprend aussi un capteur haute pression 5 placé entre le connecteur 2 et le détendeur 4, et un débitmètre 6 placé en sortie du détendeur 4. Le détendeur 4 est connecté à un circuit de sortie du gaz 7 qui permet une insufflation contrôlée du gaz par modulation de la pression à la sortie du dispositif.

Le capteur 5 et le débitmètre 6 sont reliés à un microprocesseur 8 qui contrôle des moyens d'affichage 9, avantageusement implémentés directement sur une façade du dispositif d'insufflation 1.

Le microprocesseur 8 implémente un procédé selon l'invention tel qu'illustré sur la figure 2. Ce procédé peut être déclenché manuellement par pression d'un bouton 10 placé sur la façade du dispositif d'insufflation 1 et relié au microprocesseur pour mettre en route le procédé selon l'invention ou l'être à chaque fois qu'une détente est réalisée lors de l'utilisation du dispositif 1. Dans le cas d'un déclenchement manuel, le microprocesseur 8 est avantageusement relié à une vanne du circuit de sortie 7 pour en commander le fonctionnement, son ouverture déclenchant la détente et la sortie du gaz, et/ou relié à une vanne situé dans le connecteur 2 ou à proximité de celui-ci pour également en commander le fonctionnement, son ouverture déclenchant aussi la détente et la sortie du gaz au travers du dispositif d'insufflation 1.

Le procédé selon l'invention comprend une étape E0 d'initialisation et de déclenchement d'une détente. Par exemple, deux registres du microprocesseur 8 comprenant respectivement une donnée sur la chute de pression et une donnée sur la durée écoulée pendant une détente sont initialisés à la valeur zéro.

Comme précisé ci-dessus, on note que le déclenchement de la détente peut être le fait d'une utilisation du dispositif pour une insufflation ou un déclenchement volontaire, manuelle, avant toute utilisation du dispositif pour tester la quantité de gaz restante.

Dans les deux cas, le capteur et le débitmètre envoient alors des signaux vers le microprocesseur qui les reçoit en parallèle dans les étapes E1 et E2 pendant tout le temps que dure la détente. Ces signaux sont représentatifs de la pression P en amont du détendeur, donc de la pression dans la bouteille, et du débit D en sortie du détendeur.

Dans l'exemple proposé dans la suite, on s'intéresse au cas où la détente est déclenchée par une activation manuelle des moyens d'évaluation, par exemple par pression du bouton 10.

Une telle détente est, par exemple, prédéfinie comme devant engendrer une chute de pression donnée ΔPp de 0,1 bars. Le choix de la chute de pression déclenchée par activation manuelle du procédé selon l'invention est déterminé en fonction de la quantité de gaz que l'on est prêt à perdre pour faire l'évaluation du volume après détente restant et de la sensibilité du capteur haute pression.

La pression P reçue est utilisée dans une étape E3 où est calculée la chute de pression prédéterminée ΔPp. En parallèle, dans une étape E4, le volume détendu ramené à la pression atmosphérique VA est calculé par le microprocesseur 8 et en intégrant le débit D sur le temps T écoulé lors de la détente tant que la chute de pression prédéterminée ΔPp de 0,1 bars n'est pas observée.

Dans l'exemple proposé, la détente, représentée schématiquement par la flèche du cas N rebouclant sur l'étape E1, est poursuivie jusqu'à ce que la chute de pression ΔP ait atteint la chute de pression prédéterminée ΔPp, cas O.

Dans ce cas, la détente et l'étape d'intégration E4 du débit D sur la durée T sont arrêtés et les registres sont réinitialisés dans une nouvelle étape E0.

Une telle réinitialisation permettra de recommencer l'évaluation du volume après détente restant lors d'une détente postérieure du gaz.

Cela est particulièrement utile dans le cas où la détente est due à une utilisation du dispositif en insufflation et est, donc, continue. Par exemple, dans un tel cas, des incréments de chute de pression de 0,1 bars sont utilisés pour définir des intervalles d'évaluation du volume restant V_{R}.

Suite à l'arrêt de l'intégration du volume détendu VA ramené à la pression atmosphérique P_{A}, cette valeur VA est transmise à une étape d'évaluation E5 du volume après détente restant. Cette étape E5 calcule alors le rapport du volume détendu VA sur la chute de pression ΔPp multiplié par la pression restante P2 observée par le capteur à la fin de la détente.

Ce calcul donne directement une valeur du volume après détente restant V_{R} selon les calculs théoriques présentés ci-après.

Dans le récipient sous pression, en considérant le gaz comme parfait, l'équation d'état du gaz est alors P.V=nRT, avec P la pression, V le volume du récipient, n le nombre de molécules de gaz, T la température et R la constante des gaz parfaits.

Lors de la détente ΔP du gaz de P1 à P2 au sein de la bouteille, correspondant à n1-n2 molécules sorties de la bouteille, on sait donc que V.ΔP=V(P1-P2)=(n1-n2)RT.

Comme le débitmètre mesure généralement le débit ramené à la pression atmosphérique P_{A}, le volume après détente mesuré à l'aide du débitmètre étant noté VA, on a aussi P_{A}.V_{A}=(n1-n2)RT, où VA est le volume détendu à la pression atmosphérique mesuré par le débitmètre.

On a ainsi V=P_{A}.V_{A}/ΔP.

P_{A} étant la pression atmosphérique, on constate que le rapport du volume détendu V_{A} sur la chute de pression ΔP donne une estimation du volume V du récipient 3 connecté au dispositif 1.

Il est alors possible de déterminer automatiquement le volume de la bouteille. Selon une caractéristique avantageuse, l'invention permet donc de détecter quelle est la taille du récipient 3 connecté. Cette taille est avantageusement signalée sur les moyens d'affichage 9 dont un exemple est schématiquement présenté sur la figure 3.

Cette figure 3 donne un exemple de présentation d'un écran 9, par exemple LCD, placé sur la face avant d'un dispositif d'insufflation 1 selon l'invention.

Le type de bouteille est avantageusement signalé par la mise en exergue, par exemple par l'entourage ou la mise en gras d'une des lettres S, M ou L correspondant aux différents types de bouteilles et affichées sur l'écran. Dès que le gaz dans la bouteille est en phase gazeuse, un des avantages de l'invention est donc de permettre, connaissant de manière certaine et par une mesure physique le type de bouteille connectée, de pondérer les alarmes en fonction de cela. Par exemple, le seuil en pression sera revu à la baisse pour des bouteilles de grande contenance par rapport aux bouteilles de petite contenance qui, aujourd'hui servent pour déterminer les seuils d'alarme en pression.

Les alarmes sont par exemple sonores et peuvent également être visuelles ainsi qu'illustré sur la figure 3 par l'ensemble 30 de barrettes de trois couleurs différentes, par exemple vert, orange et rouge, ou, comme illustré de plus en plus foncé de bas en haut. Le vert ou le moins foncé étant pour des pressions supérieures à 10 bars, l'orange ou le foncé moyen étant pour des pressions comprises entre 10 bars et un seuil de pression d'alarme dépendant du type de bouteille détecté et le rouge ou le foncé étant pour des pressions inférieures au seuil de pression d'alarme dépendant de la bouteille détectée.

Ensuite, le volume restant V_{R} dans la bouteille 3 est calculé en multipliant le volume V de la bouteille estimé précédemment par la pression restante P2 observée dans la bouteille 3 et mesurée par le capteur 5, à un facteur près qui est la pression P_{A}, pression à laquelle est ramené le débit mesuré par le débitmètre.

En effet, on a alors P2V=nRT=P_{A}.V_{R} où V_{R} est le volume après détente restant. P_{A} est égal à 1 dès que le débitmètre ramène ses mesures à la pression atmosphérique, ce qui est généralement le cas. On a ainsi directement accès au volume après détente restant V_{R} en multipliant le volume V déterminé précédemment par la pression P2 observée à l'issue de la chute de pression ΔP.

Le volume après détente restant V_{R} est alors aussi avantageusement affiché sur les moyens d'affichage 9. Classiquement, le débit D est aussi affiché.

En outre, il est judicieux de prévoir qu'un calcul soit en outre réalisé par le microprocesseur 8 pour estimer le temps d'insufflation restant T_{R} à l'aide du débit D tel qu'il est observé en temps réel. Pour ce dernier calcul d'estimation, il est aussi possible d'utiliser une moyenne du débit D sur un laps de temps donné. Dans l'exemple proposé, le débit D observé étant de 4 L/min et le volume restant V_{R} évalué de 120 L, le temps restant T_{R} évalué est donc de 30 minutes.

Les calculs donnés dans ce qui précède donnent une évaluation exacte du volume restant après détente lorsque le gaz est entièrement en phase gazeuse ou, lorsque celui-ci est partiellement en phase liquide mais que la détente n'a pas engendré une baisse significative de la température ayant mené à une diminution conséquente de la pression au sein du récipient.

Sinon, dans le cas où le gaz est partiellement en phase liquide, une chute de température engendre une chute de la pression qui rend les calculs précédents erronés puisque la chute de pression n'est plus, alors, due seulement à la diminution de gaz dans le récipient.

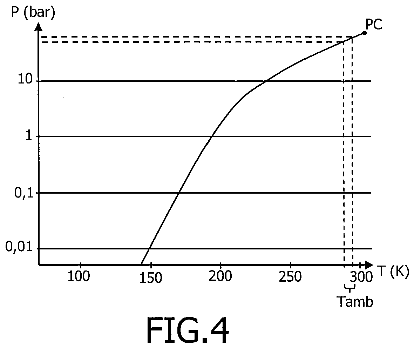

La figure 4 illustre la courbe de pression P, représentée en coordonnées logarithmiques, en fonction de la température T pour le dioxyde de carbone jusqu'à son point critique PC. On constate qu'aux températures ambiantes Tamb des zones tempérées, délimitées par les lignes pointillées, la pente en pression P n'est pas négligeable. Ainsi, la moindre variation de température T ne pourra rapidement pas être considérée comme négligeable du point de vue de la chute de pression qu'elle engendrera par rapport à la chute de pression engendrée par la sortie du gaz.

Comme la détente du gaz provoque inévitablement un refroidissement du gaz, le contrôle proposé par l'invention ne sera plus fiable.

Aussi, dans le cas où le dispositif est destiné à fonctionner avec des bouteilles où, même en fin d'utilisation, le gaz est susceptible d'être encore en phase liquide (cas des petites bouteilles), il est judicieux de munir le dispositif 1 d'une sonde de température relié au microprocesseur 8.

Le microprocesseur 8 est alors muni de moyens pour gérer une telle donnée. Il peut s'agir d'une alerte avertissant qu'au vu de la chute de température observée lors de la chute de pression, le volume restant affiché est nécessairement erroné. Le microprocesseur 8 peut également être tel que, dans le cas où il détecte une chute de température supérieure à, par exemple 1°C, pendant la détente servant au calcul du volume restant, celui-ci ne soit pas affiché.

Enfin, dans une réalisation plus précise, le microprocesseur peut être tel qu'il est apte à déterminer un facteur correctif calculé en fonction des modifications de température observées par la sonde de température. Ce facteur correctif est alors introduit dans le calcul du volume restant. Il sera adapté en fonction du gaz utilisé en se référant à la courbe pression / température du gaz considéré.

On remarque enfin que diverses mises en oeuvre peuvent être réalisées selon les principes de l'invention définie par les revendications qui suivent. Notamment, le procédé selon l'invention peut être mis en oeuvre dès qu'une détente est effectuée. Le procédé peut ainsi être réalisé en continu avec des rafraichissements périodiques du calcul et de l'affichage du volume après détente restant, les rafraichissements étant avantageusement faits à des intervalles de chute de pression réguliers.

Il est également possible de prévoir l'émission d'un son lorsque le volume restant apparaît inférieur à une valeur donnée. Une telle alarme sonore peut être implémentée seule ou en combinaison avec des moyens d'affichage tels que présentés ci-dessus.

## Revendications

1. Dispositif médical (1) d'insufflation de gaz comprenant, au moins, un circuit de sortie (7) du gaz, un connecteur (2) pour connecter un récipient sous pression (3) dans lequel le gaz est conditionné, un détendeur (4) placé entre le connecteur (2) et le circuit de sortie (7) du gaz, un capteur haute pression (5) placé entre le connecteur (2) et le détendeur (4), un débitmètre (6) placé en sortie du détendeur (4), et un microprocesseur (8) relié au moins au capteur haute pression (5) pour recevoir des signaux représentatifs de la pression observée au niveau du connecteur (2) et au débitmètre (6) pour recevoir des signaux représentatifs du débit en sortie du détendeur (4), **caractérisé en ce que** le microprocesseur (8) comprend des moyens pour évaluer un volume de gaz après détente restant (V_{R}) en fonction des signaux reçus en provenance du capteur haute pression (5) et du débitmètre (6) lors d'une détente du gaz, ces moyens d'évaluation comportant des moyens pour évaluer le volume de gaz après détente restant (V_{R}) comme étant le rapport du volume détendu (V_{D}) obtenu à partir du débit (D) mesuré par le débitmètre (6) pendant la détente et de la durée (T) de celle-ci sur la chute de pression (ΔP) engendrée par cette détente et mesurée au niveau du capteur haute pression (5) multiplié par la pression restante (P) mesurée par le capteur haute pression (5) au terme de la détente.

2. Dispositif (1) selon la revendication 1, **caractérisé en ce que** les moyens d'évaluation comportent des moyens de calcul du volume détendu à partir du débit observé et de la durée de la détente au terme de la chute d'un intervalle de pression choisi préalablement.

3. Dispositif (1) selon la revendication 1, **caractérisé en ce que** les moyens d'évaluation comportent des moyens de mesure d'une chute de pression au terme de la détente d'un volume détendu choisi préalablement.

4. Dispositif (1) selon l'une des revendications 2 et 3, **caractérisé en ce que**, lors de l'utilisation du dispositif (1), les moyens de calcul du volume détendu ou les moyens de mesure de la chute de pression sont tels que le calcul ou la mesure sont effectués respectivement à chaque chute d'un intervalle de pression choisi ou au terme de chaque détente successive d'un volume détendu choisi.

5. Dispositif (1) selon l'une des revendications précédentes, **caractérisé en ce qu'**il inclut une sonde de température apte à mesurer la température du gaz sortant du récipient (3) et relié au microprocesseur (8), le microprocesseur (8) étant apte à utiliser cette mesure de température pour assurer la fiabilité du volume de gaz après détente restant évalué.

6. Dispositif (1) selon la revendication 5, **caractérisé en ce que** le microprocesseur (8) comprend des moyens de déclenchement pour déclencher, lorsqu'une modification de la température supérieure à une limite donnée est observée lors d'une détente servant à l'évaluation du volume après détente restant, une action choisi parmi les suivantes : alerte, détermination d'un facteur correctif en fonction de la modification de température et utilisation de ce facteur correctif dans l'évaluation du volume après détente restant.

7. Dispositif (1) selon l'une des revendications précédentes, **caractérisé en ce que** les moyens d'évaluation sont agencés de telle sorte qu'ils sont activés lorsque le capteur haute pression (5) mesure une pression inférieure à un seuil donné.

8. Dispositif (1) selon l'une des revendications précédentes, **caractérisé en ce qu'**en dehors de l'utilisation du dispositif (1), les moyens d'évaluation sont agencés de telle sorte qu'ils sont activables ponctuellement par déclenchement d'une détente prédéfinie par une chute de pression donnée ou par un volume de gaz détendu donné.

9. Dispositif (1) selon la revendication 8, **caractérisé en ce qu'**il comprend des moyens d'activation ponctuelle des moyens d'évaluation manuels ou fonctionnant par réception d'un signal.

10. Dispositif (1) selon la revendication 9, **caractérisé en ce que** les moyens manuels d'activation sont constitués par un bouton (10) placé sur le dispositif (1) et destiné à être pressé par un opérateur ou utilisateur.

11. Dispositif (1) selon la revendication 9, **caractérisé en ce qu'**il comprend des moyens pour recevoir un signal provenant d'une télécommande ou un signal vocal.

12. Dispositif (1) selon l'une des revendications précédentes, **caractérisé en ce qu'**il comprend des moyens d'affichage (9) reliés au microprocesseur (8) pour afficher le volume de gaz après détente restant.

13. Dispositif (1) selon l'une des revendications précédentes, **caractérisé en ce que** les moyens d'évaluation comprennent des moyens permettant la détection du type de récipient (3) connecté en fonction du calcul du rapport du volume détendu obtenu à partir du débit et de la durée de celle-ci sur la chute de pression engendrée par la détente et mesurée au niveau du capteur haute pression (5).

14. Dispositif (1) selon la revendication 13, **caractérisé en ce qu'**il comprend des moyens d'affichage (9) pour afficher le type de récipient connecté détecté.

15. Procédé apte à être implémenté dans un microprocesseur (8) d'un dispositif (1) d'insufflation selon l'une des revendications 1 à 14, pour évaluer un volume de gaz après détente restant lors d'une détente du gaz contenu dans un récipient sous pression (3) connecté au dispositif d'insufflation (1), comprenant les étapes de:
- déclenchement d'une détente,
- réception de signaux représentatifs de la pression au sein du récipient (3) en provenance du capteur haute pression (5),
- réception de signaux représentatifs du débit en sortie du détendeur (4) en provenance du débitmètre (6), et
- évaluation du volume de gaz après détente restant (V_{R}) comme étant le rapport du volume détendu (V_{D}) obtenu à partir du débit (D) mesuré par le débitmètre (6) pendant la détente et de la durée (T) de celle-ci sur la chute de pression (ΔP) engendrée par cette détente et mesurée au niveau du capteur haute pression (5) multiplié par la pression restante (P) mesurée par le capteur haute pression (5) au terme de la détente.

16. Support d'enregistrement lisible par un ordinateur sur lequel est enregistré un programme d'ordinateur comprenant des instructions pour l'exécution des étapes du procédé selon la revendication 15.

## Patentansprüche

1. Medizinische Vorrichtung (1) zum Gaseinblasen, wenigstens umfassend einen Gasausgangskreis (7), einen Verbinder (2) zum Verbinden eines Druckbehälters (3), in dem das Gas enthalten ist, einen Druckminderer (4), der zwischen dem Verbinder (2) und dem Gasausgangskreis (7) angeordnet ist, einen Hochdrucksensor (5), der zwischen dem Verbinder (2) und dem Druckminderer (4) angeordnet ist, einen Durchflussmesser (6), der am Ausgang des Druckminderers (4) angeordnet ist, und einen Mikroprozessor (8), der wenigstens mit dem Hochdrucksensor (5) verbunden ist, um Signale, die für den im Bereich des Verbinders (2) festgestellten Druck repräsentativ sind, zu empfangen, sowie mit dem Durchflussmesser (6), um Signale, die für den Durchsatz am Ausgang des Druckminderers (4) repräsentativ sind, zu empfangen, **dadurch gekennzeichnet, dass** der Mikroprozessor (8) Mittel zum Auswerten eines Restgasvolumens nach Entspannung (V_{R}) in Abhängigkeit von den von dem Hochdrucksensor (5) und von dem Durchflussmesser (6) empfangenen Signalen während einer Entspannung des Gases umfasst, wobei diese Auswertungsmittel Mittel umfassen, um das Restgasvolumen nach Entspannung (V_{R}) als das Verhältnis des entspannten Volumens (V_{D}), das anhand des durch den Durchflussmesser (6) während der Entspannung gemessenen Durchsatzes (D) und deren Dauer (T) über den durch diese Entspannung erzeugten und im Bereich des Hochdrucksensors (5) gemessenen Druckabfall (ΔP) erhalten wird, multipliziert mit dem Restdruck (P), welcher durch den Hochdrucksensor (5) am Ende der Entspannung gemessen wird, auszuwerten.

2. Vorrichtung (1) nach Anspruch 1, **dadurch gekennzeichnet, dass** die Auswertungsmittel Mittel zur Berechnung des entspannten Volumens anhand des festgestellten Durchsatzes und der Dauer der Entspannung am Ende des Abfalls eines zuvor gewählten Druckintervalls umfassen.

3. Vorrichtung (1) nach Anspruch 1, **dadurch gekennzeichnet, dass** die Auswertungsmittel Mittel zum Messen eines Druckabfalls am Ende der Entspannung eines zuvor gewählten entspannten Volumens umfassen.

4. Vorrichtung (1) nach einem der Ansprüche 2 und 3, **dadurch gekennzeichnet, dass** während der Verwendung der Vorrichtung (1) die Mittel zur Berechnung des entspannten Volumens oder die Mittel zum Messen des Druckabfalls derart sind, dass die Berechnung oder die Messung bei jedem Abfall eines gewählten Druckintervalls bzw. am Ende einer jeden nachfolgenden Entspannung eines gewählten entspannten Volumens durchgeführt werden.

5. Vorrichtung (1) nach einem der vorhergehenden Ansprüche, **dadurch gekennzeichnet, dass** sie eine Temperatursonde enthält, die geeignet ist, die Temperatur des den Behälter (3) verlassenden Gases zu messen, und die mit dem Mikroprozessor (8) verbunden ist, wobei der Mikroprozessor (8) geeignet ist, diese Temperaturmessung zu verwenden, um die Zuverlässigkeit des ausgewerteten Restgasvolumens nach Entspannung sicherzustellen.

6. Vorrichtung (1) nach Anspruch 5, **dadurch gekennzeichnet, dass** der Mikroprozessor (8) Auslösemittel umfasst, um dann, wenn eine Änderung der Temperatur oberhalb einer gegebenen Grenze während einer Entspannung, die zur Auswertung des Restvolumens nach Entspannung dient, festgestellt wird, eine Aktion auszulösen, die aus den folgenden ausgewählt ist: Alarm, Bestimmung eines Korrekturfaktors in Abhängigkeit von der Temperaturänderung und Verwendung dieses Korrekturfaktors bei der Auswertung des Restvolumens nach Entspannung.

7. Vorrichtung (1) nach einem der vorhergehenden Ansprüche, **dadurch gekennzeichnet, dass** die Auswertungsmittel derart eingerichtet sind, dass sie dann aktiviert werden, wenn der Hochdrucksensor (5) einen Druck unterhalb einer gegebenen Schwelle misst.

8. Vorrichtung (1) nach einem der vorhergehenden Ansprüche, **dadurch gekennzeichnet, dass** außerhalb der Verwendung der Vorrichtung (1) die Auswertungsmittel derart eingerichtet sind, dass sie durch Auslösen einer durch einen gegebenen Druckabfall oder durch ein gegebenes entspanntes Gasvolumen vordefinierten Entspannung punktuell aktivierbar sind.

9. Vorrichtung (1) nach Anspruch 8, **dadurch gekennzeichnet, dass** sie manuelle oder durch Empfang eines Signals funktionierende Mittel zur punktuellen Aktivierung der Auswertungsmittel umfasst.

10. Vorrichtung (1) nach Anspruch 9, **dadurch gekennzeichnet, dass** die manuellen Aktivierungsmittel durch einen Knopf (10), welcher an der Vorrichtung (10) angeordnet und dazu bestimmt ist, durch eine Bedienungsperson oder einen Benutzer gedrückt zu werden, gebildet sind.

11. Vorrichtung (1) nach Anspruch 9, **dadurch gekennzeichnet, dass** sie Mittel zum Empfangen eines Signals von einer Fernsteuerung oder eines Sprachsignals umfasst.

12. Vorrichtung (1) nach einem der vorhergehenden Ansprüche, **dadurch gekennzeichnet, dass** sie Anzeigemittel (9) umfasst, die mit dem Mikroprozessor (8) verbunden sind, um das Restgasvolumen nach Entspannung anzuzeigen.

13. Vorrichtung (1) nach einem der vorhergehenden Ansprüche, **dadurch gekennzeichnet, dass** die Auswertungsmittel Mittel umfassen, die die Erfassung der Art von angeschlossenem Behälter (3) in Abhängigkeit von der Berechnung des Verhältnisses des entspannten Volumens, das anhand des Durchsatzes und der Dauer der Entspannung über den durch die Entspannung erzeugten und im Bereich des Hochdrucksensors (5) gemessenen Druckabfall erhalten wird, ermöglichen.

14. Vorrichtung (1) nach Anspruch 13, **dadurch gekennzeichnet, dass** sie Anzeigemittel (9) umfasst, um die erfasste Art von angeschlossenem Behälter anzuzeigen.

15. Verfahren, das geeignet ist, in einen Mikroprozessor (8) einer Einblasvorrichtung (1) nach einem der Ansprüche 1 bis 14 implementiert zu werden, um ein Restgasvolumen nach Entspannung während einer Entspannung des Gases, das in einem an die Einblasvorrichtung (1) angeschlossenen Druckbehälter (3) enthalten ist, auszuwerten, umfassend die Schritte des:
- Auslösens einer Entspannung,
- Empfangens von Signalen, die für den Druck innerhalb des Behälters (3) repräsentativ sind, von dem Hochdrucksensor (5),
- Empfangs von Signalen, die für den Durchsatz am Ausgang des Druckminderers (4) repräsentativ sind, von dem Durchflussmesser (6), und
- Auswertens des Restgasvolumens nach Entspannung (V_{R}) als das Verhältnis des entspannten Volumens (V_{D}), das anhand des durch den Durchflussmesser (6) während der Entspannung gemessenen Durchsatzes (D) und deren Dauer (T) über den durch diese Entspannung erzeugten und im Bereich des Hochdrucksensors (5) gemessenen Druckabfall (ΔP) erhalten wird, multipliziert mit dem Restdruck (P), welcher durch den Hochdrucksensor (5) am Ende der Entspannung gemessen wird.

16. Durch einen Computer lesbarer Aufzeichnungsträger, auf dem ein Computerprogramm gespeichert ist, das Befehle für die Ausführung der Schritte des Verfahrens nach Anspruch 15 umfasst.

## Claims

1. A medical gas insufflation device (1) comprising at least a gas outlet circuit (7), a connector (2) for connecting a pressurized vessel (3) in which the gas is packaged, an expander (4) placed between the connector (2) and the gas outlet circuit (7), a high-pressure sensor (5) placed between the connector (2) and the expander (4), a flow meter (6) placed at the outlet from the expander (4), and a microprocessor (8) connected at least to the high-pressure sensor (5) to receive signals representative of the pressure observed at the connector (2) and to the flow meter (6) to receive signals representative of the flow rate at the outlet from the expander (4), the device being **characterized in that** the microprocessor (8) comprises evaluation means for evaluating the remaining volume of gas after expansion (V_{R}) as being the ratio of the expanded volume (V_{D}) obtained from the flow rate (D) measured by the flow meter (6) during the expansion and the duration (T) of the expansion divided by the pressure drop (ΔP) generated by the expansion and measured by the high-pressure sensor (5), multiplied by the remaining pressure (P) as measured by the high-pressure sensor (5) at the end of the expansion operation.

2. A device (1) according to claim 1, **characterized in that** the evaluation means comprise means for calculating the expanded volume from the observed flow rate and from the duration needed by the expansion operation to cause the pressure to drop by a previously-selected interval.

3. A device (1) according to claim 1, **characterized in that** the evaluation means comprise means for measuring a pressure drop at the end of the operation of expanding of a previously-selected expanded volume.

4. A device (1) according to claim 2 or claim 3, **characterized in that**, while the device (1) is in use, the means for calculating the expanded volume or the means for measuring the pressure drop are such that the calculation or the measurements are performed respectively on each occasion the pressure drops by the selected interval or at the end of each successive occasion on which a selected expanded volume has been expanded.

5. A device (1) according to any preceding claim, **characterized in that** it comprises a temperature probe suitable for measuring the temperature of the gas leaving the cylinder (3) and connected to the microprocessor (8), the microprocessor (8) being suitable for using said temperature measurement to ensure the reliability of the evaluated remaining volume of gas after expansion.

6. A device (1) according to claim 5, **characterized in that** the microprocessor (8) comprises means for triggering an action when a temperature change greater than a given limit is observed during an expansion operation used for evaluating the remaining volume after expansion, the action being selected from the following: issuing a warning, determining a correction factor as a function of the temperature change and using said correction factor when evaluating the volume remaining after expansion.

7. A device (1) according to any preceding claim, **characterized in that** the evaluation means are arranged in such a manner that they are activated when the high-pressure sensor (5) measures a pressure below a given threshold.

8. A device (1) according to any preceding claim, **characterized in that** when the device (1) is not in use, the evaluation means are arranged in such a manner that they can be activated momentarily by triggering an expansion operation that is predefined by a given pressure drop or by a given volume of expanded gas.

9. A device (1) according to claim 8, **characterized in that** it comprises means for momentarily activating the evaluation means manually or that operate on receiving a signal.

10. A device (1) according to claim 9, **characterized in that** the manual activation means are constituted by a button (10) placed on the device (1) so as to be pressed by an operator or a user.

11. A device (1) according to claim 9, **characterized in that** it comprises means for receiving a signal coming from a remote control or a voice signal.

12. A device (1) according to any preceding claim, **characterized in that** it comprises display means (9) connected to the microprocessor (8) to display the remaining volume of gas after expansion.

13. A device (1) according to any preceding claim, **characterized in that** the evaluation means comprise means enabling the type of vessel (3) connected thereto to be detected as a function of calculation of the ratio of the expanded volume obtained from the flow rate and the duration of the expansion operation divided by the pressure drop caused by the expansion and measured by the sensor (5).

14. A device (1) according to claim 13, **characterized in that** it comprises display means (9) for displaying the detected type of vessel that is connected thereto.

15. A method suitable for being implemented in a microprocessor (8) of an insufflation device (1) according to any one of claims 1 to 14, in order to evaluate a remaining volume of gas after expansion during an operation of expanding the gas contained in a vessel (3) connected to the insufflation device (1), the method comprising the steps of:
• triggering an expansion operation;
• receiving signals representative of the pressure within the vessel (3) and coming from the high-pressure sensor (5);
• receiving signals representative of the flow rate at the outlet from the expander (4) and coming from the flow meter (6); and
• evaluating the remaining volume of gas after expansion (V_{R}) as being the ratio of the expanded volume (V_{D}) obtained from the flow rate (D) measured by the flow meter (6) during the expansion and the duration (T) of the expansion divided by the pressure drop (ΔP) generated by the expansion and measured by the high-pressure sensor (5), multiplied by the remaining pressure (P) as measured by the high-pressure sensor (5) at the end of the expansion operation.

16. A computer-readable recording medium having a computer program recorded thereon including instructions for executing the steps of the method according to claim 15.
